# EUROPEAN PATENT SPECIFICATION

(11) **EP 0 310 205 B1**
(45) Date of publication and mention of the grant of the patent: **20.07.1994**
(21) Application number: 88202348.4
(22) Date of filing: 20.03.1985
(51) Int. Cl.: B01D 63/16, B01D 65/08, B01D 35/22, A61M 1/34

(54) **Filtering a liquid suspension**
Filtration einer flüssigen Suspension
Filtration d'une suspension liquide

(30) Priority: 21.03.1984 US 591925
(43) Date of publication of application: 05.04.1989
(62) Divisional of application: 85901785.7
(73) Proprietor: McLaughlin, William Francis, Newport Beach California 92663 (US)
(72) Inventor: Schoendorfer, Donald W., Santa Ana, CA 92705 (US)
(74) Representative: Smith, Philip Antony

(56) References cited:
- WO-A-85/02783
- DE-A- 2 812 042
- FR-A- 2 326 958
- GB-A- 1 480 406
- US-A- 3 396 103
- US-A- 3 581 900
- US-A- 4 212 741
- ESCHER WYSS MITTEILUNGEN, 2/1978-1/1979, pages 21-23; W. TOBLER: "Dynamische Filtration - das apparative Konzept des Escher Wyss-Druckfilters"
- ESCHER WYSS MITTEILUNGEN, 2/1978-1/1979, pages 24-30; J. LIEBERHERR:"Hydrodynamik der Ringspaltströmung zwischen permeablen Zylinderwänden"

## Description

This invention relates to membrane filtration of matter from liquid suspensions, and particularly to biomedical applications of such technology. It is especially relevant to, but not limited to, the separation or fractionation of the constituents of blood.

Techniques for the separation and collection of given constituents of whole blood are in wide use for many therapeutic, medical and experimental applications. Plasmapheresis (the separation of fluid plasma from the red and white cells of whole blood) forms the basis of widespread plasma storage and supply systems, and also is employed increasingly in therapeutic apheresis. Plasma is collected from individual donors by withdrawing whole blood, separating the plasma, and preferably returning high hematocrit (high cell percentage) fractions back to the donor. Plasmapheresis by centrifugal separation is widely used in laboratories but is essentially a batch process of limited convenience and commercial practicality. Continuous centrifugal separation is desired if plasma is to be collected rapidly and with minimum donor inconvenience, and in the modern state of the art this cannot be done at reasonable cost. Blood handling and collection systems must be completely sterile, which in effect requires that all elements in contact with the blood be low cost disposable components or devices. Many workers in the art have thus experimented with membrane filtration techniques, in which a membrane with suitably small pore size (e.g. 0.5 microns) is utilized to filter plasma from the blood. Because of the viscous and complex quality of whole blood, simple filtration does not suffice because deposition (clogging of pores with cellular matter) quickly decreases the efficiency of transfer through the membrane.

Recognizing these problems, a number of workers in the art have sought to utilize the shear principle so as to increase efficiency. Transport of whole blood laterally across a membrane surface which is moving relative to an opposed surface sets up shearing forces on the blood sheet, tending to keep the cellular matter in motion and to lift it away from the membrane pores, substantially reducing the deposition problem. Workers in the art have observed a generally increasing relationship between the amount of shear and the efficiency of the filtration process, with an upper limit being imposed by unwanted cell disruption or hemolysis, typically at maximum shear rates of 7,500 to 10,000/sec with prior devices.

Membrane filtration effectively appeared to have reached a practical limit with various flat membrane configurations, because of various pressure losses and flow discontinuities. In practice, a substantial membrane area has been required for such configurations, in order to enable plasma collection at a reasonable rate from an individual donor. However the membrane cost is high and the system efficiency decreases with the duration of usage. Thus the desirable objective of a low cost disposable system has not been heretofore achieved with a reliably operating system.

More recently, however, a remarkable advance in blood separation technology using membrane filtration has arisen from a different structure, described in European Patent Specification EP-A-0112152 filed 13.12.83, published 27.6.84 and claiming a priority of 13.12.82 (referred to below by the name of the inventor - Fischel). The configuration described in that patent application provides filtration rates in excess of ten times that found in prior membrane filtration devices, for a given surface area. A membrane covered spinner, having an interior collection system, is disposed within a stationary shell, and blood is fed into the space between the spinner and the shell, moving both circumferentially about the shell and along the longitudinal axis to a spaced apart exit region. A practical device, having a gap of .030" (.076cm.) and a rotational velocity of approximately 3600 r.p.m., with a spinner diameter of 1" (2.54 cm) and length of 3" (7.5 cm) enables plasma to be derived at approximately 45 ml/min, and with high plasma recovery (e.g. in excess of 70%). A plasma recovery of 0.9 ml/cm²/min is achieved in contrast to prior art flat plate systems providing about 0.039 ml/cm²/min and hollow fibre systems providing 0.013 ml/cm²/min. The significant improvement in filtration efficiency thus afforded makes a low cost plasmapheresis disposable practical for the first time, and enables two to three units of blood to be transferred conveniently and quickly as high hematocrit remainder is returned to the donor.

While flow conditions existing between a rotating spinner and a concentric shell have been much studied, being termed Couette flow, the extraction of a filtrate through a membrane on the spinner represents a special case of potentially wide applicability. Generically, this configuration encompasses a number of systems in which a filter member spinning within a bath is used to prevent or limit particulates in the bath from adhering to the filter, while drawing filtrate into the interior of the spinner. Particular examples of these are shown in an article by M. Lopez-Leiva entitled "Ultrafiltration at Low Degrees of Concentration Polarization: Technical Possibilities" in Desalination (Netherlands) Vol. 35, pp. 125-128 (1980) dealing with the concentration of milk products, and in US-A-4,184,952 (Shell Oil) dealing with the extraction of oil from basic sediment and water. However, there is nothing in these disclosures that would tend to indicate that the significant improvement achieved by Fischel in plasmapheresis would even be possible, or explain the mechanism of separation in a such system. The Fischel patent application as filed hypothesized that a "shear centrifugation" effect takes place, with centrifugal forces acting to cause migration of the cellular matter outwardly toward the stationary wall, while a plasma-rich layer resides at the surface. Limiting factors on the performance of this system were described in terms of conditions to maintain laminar flow between the spinner and the outer wall, while also exerting sufficient centrifugal force to achieve outward cell migration. Thus the application purported to distinguish from other rotating flow systems in which relative movement between two concentric cylinders causes creation of localized cellular structures, called Taylor vortices, between the walls.

Taylor vortices also have been intensively investigated in the literature and a number of devices, particularly oxygenators proposed by Brumfield, in US-A-3,771,658, 3,771,899 and 4,212,741, have been considerd that utilize such effects. Most of the investigations of Taylor vortices are concerned with theoretical aspects, and few systems, including the oxygenators, have been successfully implemented using these principles. No systems using Taylor vortices are known in which the dynamics of the fluid medium between the members are affected or altered by continuous extraction of constituents from the medium.

The situation in which a filtrate is extracted from a complex fragile living system, such as whole blood, can be seen to involve many complex factors. It is shown hereafter that the operating mode in the Fischel system does not create laminar flow under the conditions stated above, and that the withdrawal of the plasma itself generates forces substantially in excess of the centrifugal forces acting on the blood cells. While the particular plasmapheresis system of Fischel functions with the efficiency described, further improvements as to the limits and optimums of the process are made feasible by the use of configurations and operating conditions which take into account all of the dominant system requirements to establish and enhance a substantially different mode of operation.

An article by W.Tobler in "Escher Wyss Mitteilungen", 2/1978 - 1/1979, at pages 21 to 23, describes a system for filtering a liquid suspension, comprising a housing having an inner surface carrying a filter membrane and a rotor mounted for rotation within the housing and having an outer surface which together with the inner surface of the housing defines a gap to receive the liquid suspension. In that filter the rotor carries a filter membrane on its outer surface and filtrate passes into the interior of the rotor to flow out at the lower end. The housing comprises an annular chamber into which filtrate flows through the filter membrane and from which the filtrate then flows out of the apparatus.

The filtering system of the present invention is characterized in that the housing is constructed of separable parts, each having two end portions, the end portions providing end bearings for the rotor, and retaining means releasably holding the parts against radial separation, the inner surface of the housing has a network of surface grooves, each of the separable parts has a corresponding filter membrane releasably attached to its inner surface and covering the surface grooves, and a passage is provided in the housing which communicates with the surface grooves when the separable parts are joined and thus receives the filtrate passing through the filter membrane.

US-A-4,212,741 describes blood processing apparatus with a cylindrical rotor which carries a semi-permeable membrane and is disposed within a split housing formed in two halves. The present invention does not merely improve access to the interior of the filter but enables removal and replacement of the membranes.

The present application is divided out of European Patent Application No. 85901785.7 (EP-B-0177564). For a more detailed discussion of the flow phenomena which occur in systems in accordance with the present invention reference is made to EP-B-0177564. In summary, an operating regime is established, in Couette-type flow, in which the radial gap, spinner diameter, and angular velocity are selected for a given liquid suspension to establish strong but controlled Taylor vortices along the length of the spinner. The strong vortex action creates a series of adjacent annular vortex cells about the spinner and moving along the length of the spinner while alternating in direction of internal circulation. The vortex action is strong enough for the cells to substantially fill the radial gap, and matter in suspension thus is impelled in a complex path having orthogonal velocity components at both the spinner and outer wall surfaces. Matter may be filtered via a surface membrane from both the inner (spinner) surface and the outer (stationary wall) surface, or the outer surface alone. The velocity components at the membrane surface contribute meaningfully to the high shear rate established by relative spinner-wall motion and provide an interior sweeping motion within the radial gap that tends to clear the membrane of matter that would otherwise deposit on the membrane pores as filtrate is rapidly extracted.

Systems and methods in accordance with the invention are particularly useful in overcoming the many and difficult problems of hemapheresis systems, but are equally well suited for a wide range of other applications. The concept appears useful wherever the agregate viscosity of the system permits establishment of strong Taylor vortices over a length of spinner despite constant filtrate extraction, and the density of solid or particulate matter within the suspension allows entrainment of the matter within the circulating vortices.

A feature of systems in accordance with the invention is that the surface topology of the membrane is selected relative to the nature of the suspension being filtered. To enhance the vortex action and minimize occlusion of membrane by blood, for example, a smooth surfaced membrane is employed that has surface irregularities no greater than the pore size. Despite the fact that membrane surface variations may be minute in many commercial membranes, they nonetheless can hemolyze and entrap red cells while diminishing the local surface effects of vortex action. Thus superior results are achieved by employing smooth surfaced membranes under these conditions.

Systems in accordance with the invention implant the filter membrane in the outer, stationary, wall with a number of constructional advantages and minimal reduction in operating efficiency. The stationary membrane surfaces may readily be replaced for use of the system as a separator for diagnostic applications, or for applications where the system is to be operated continuously for extended periods. In this type of separator, the vortex flow is established by a spinner retained within a concentric split housing that can be opened to replace longitudinal filter membranes. An external magnetic drive rotates the spinner at an angular velocity that insures, relative to the gap and suspension viscosity, that strong vortices exist to provide sweeping action and freedom from clogging at the membrane. With a slightly lower extraction rate than used in an interior membrane system operating with a given spinner surface velocity, a high % take is nonetheless achieved. The velocity or gap dimension can be increased to provide a higher % take in many instances. The system has further advantages if used for diagnostic or analytical purposes because the membrane can be replaced and the unit can repeatedly be reused by rinsing the membrane between operations.

A specific example of a system for providing superior plasmapheresis operation employs maximized gap spacings for a given rotational rate, together with Taylor numbers in the range of 70 to 250 (preferably 100 to 250) and shear rates of 7500/sec to 10000/sec maximum. Among the further aspects of the invention, pore sizes can be used that are in the range of 0.1 to 1.0 microns, these being larger and more efficient than those heretofore used. In addition, blood flow through the separation device can be against gravity if desired for specific purposes. Inasmuch as minimal membrane area is desired for low cost plasmapheresis disposals, a relatively small range of gap sizes and angular velocities is employed for achieving maximized and constant throughput rates for plasma. For example, with a 1" (2.54cm) diameter rotor the gap dimension is held in the range between about 0.018" (0.046cm) and 0.030" (0.076cm) for rotor angular velocities of 3000 to 3600 r.p.m.

### Brief Description of the Drawings

A better understanding of the invention may be had by reference to the following description, taken in conjunction with the accompanying drawings, in which:
Fig. 1 is a combined perspective view, partially broken away and block diagram of a plasmapheresis system in accordance with the invention of EP-B-0177564;
Fig. 2 is an enlarged and simplified fragmentary perspective view of a part of the plasma separation device in the arrangement of Fig. 1, showing vortex flow characteristics;
Fig. 3 is an enlarged side sectional view of the arrangement of Fig. 2, depicting vortex flow as described in the literature and as seen in operation;
Fig. 4 is a graph showing changes in plasma delivery for given input flow, with changes in the relationship between gap and angular velocity for a given size spinner;
- Fig. 5 is a graph showing plasma flux changes for different gap sizes, other factors remaining constant;
Fig. 6 is a perspective view, partially broken away, of a system in accordance with the invention utilizing a stationary membrane;
Fig. 7 is a side sectional view of the system of Fig. 6; and
Fig. 8 is a perspective view, somewhat idealized and not to scale, of a fragment of a filtration membrane, showing the surface irregularities in one type of membrane.

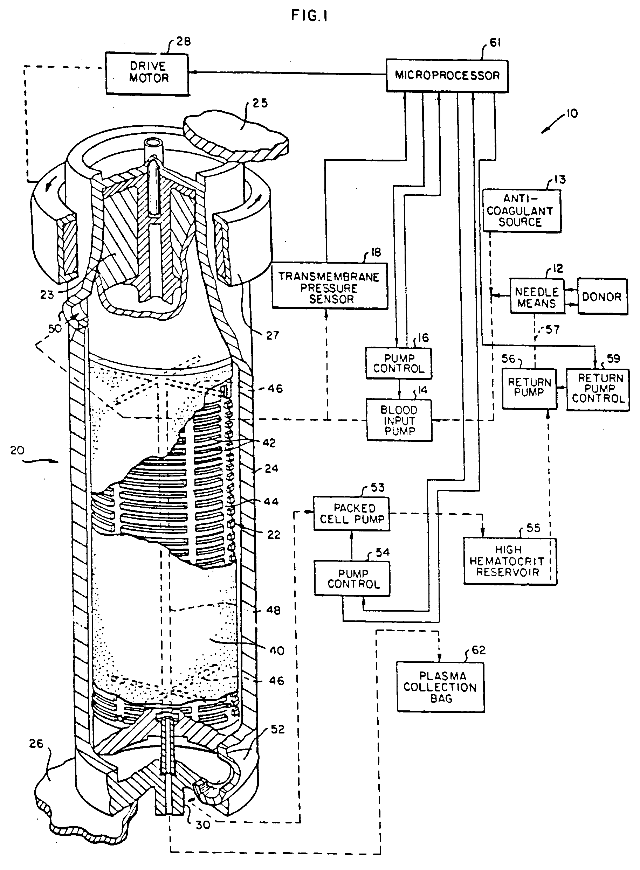

A plasmapheresis system 10, referring now to Fig. 1, in which the elements have been depicted only generally, provides a particularly suitable example of a blood separation system. Whole blood is taken from a donor via a needle means 12, shown as a single needle although a double needle system may alternatively be used. Disposable tubing is utilized to conduct the blood from the donor, and to combine it with a flow of anticoaqulant from a source 13 (flow control for the anticoagulant being of any one of a number of known types and therefore not shown). An input blood pump 14, such as a peristaltic or pressure roller device, feeds the combined flow, when actuated by an associated blood pump control 16, to a transmembrane pressure sensor 18 and also to a disposable plasma separator device 20. The plasma separator 20 is in the form of a spinner 22 having magnetic elements 23 integral with one end and rotatable about a central longitudinal axis within a stationary housing or shear wall 24. The spinner 22 is receivable between a pair of positioning supports 25, 26 spaced apart along the central axis, and shown only generally. The upper support 25, seen only in fragmentary form, provides a positioning seat for a non-rotating upper portion of the separator device 20. At the upper end also a magnetic drive 27 (not shown in detail) encompassing and magnetically coupling to the magnetic elements 23 integral with the spinner 22, is rotated by a drive motor 28. The lower support 26 receives the lower end of the stationary housing 24 and defines an opening through which a plasma outlet 30 coaxial with the central axis may provide plasma as output.

The surface of the spinner 22 may be covered by a filter membrane 40 of a type conventionally used in blood filtration, and having surface apertures in the range of 0.1 to 1.0 microns. In the present system, however, substantial advantages are obtained by using membranes having particular physical characteristics and a pore size in the range of 0.8 to 1.0 microns, as described below. Under the membrane 40, the spinner surface is configured to define a plurality of circumferential grooves 42, interconnected by longitudinal grooves 44 which in turn communicate via radial conduits 46 with a central manifold 48. The manifold 48 is in communication, through an end seal and bearing arrangement (not shown in detail) with the plasma outlet 30.

While blood from the donor is fed into the space between the spinner 22 and inner wall of the concentric housing 24 via a tangential blood inlet 50 coupled by a flexible tubing (not shown in detail) to the blood input pump 16. A high hematocrit return flow is taken from a tangential outlet orifice 52 spaced apart from the inlet along the longitudinal axis of the separator device 20. Flexible tubing (also not shown in detail) couples the outlet 52, through a peristaltic packed cell pump 53 operated by a control 54, to a high hematocrit reservoir 55. Separator 20 operation can thereby be isolated from the donor so that alternate pump and return cycles can be used with a single needle device. Packed cells are rein fused in the donor at the needle means by a return pump 56 in a return line 57 between the needle means 12 and the reservoir 55. A return pump control 59 operates the return pump 56 at rates and times determined by the control system, which may include means (not shown) for sensing the level in the reservoir 55.

The separator device 20, the mechanical operation of which is described in greater detail in the previously mentioned Fischel application, extracts plasma from the whole blood flow, through the membrane 40. The plasma flows through the membrane 40 into the circumferential and longitudinal grooves 42, 44 on the spinner 22 surface and then into the central manifold 48 via the radial conduits 46. The collected plasma in the central manifold 48 passes through the plasma outlet 30 to a plasma collection bag 62. The typical donor supplies two to three units of plasma in thirty to forty-five minutes, this being a rate consistent with blood supply from and high hematocrit return to the donor, without discomfort or substantial danger. As noted in the Fischel application, the rate of extraction remains Substantially constant. Under proper operation, the plasma is clear and golden in color, being essentially completely free of cell damage and consequent hemolysis.

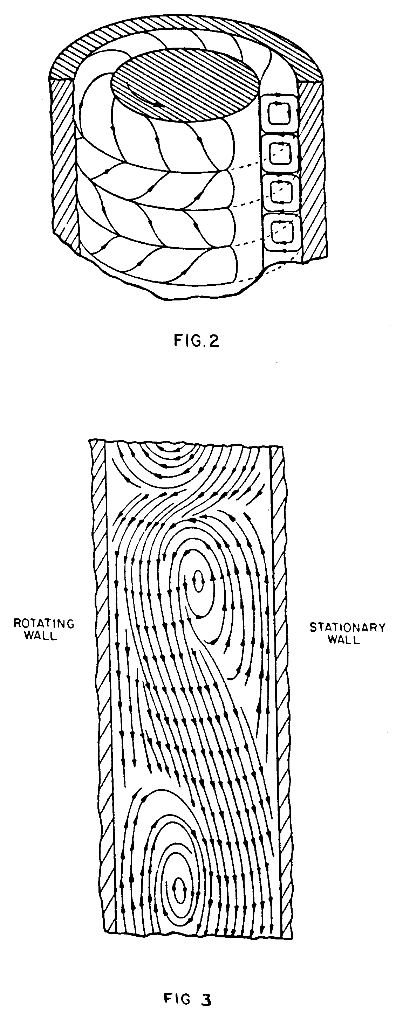

It is, however, extremely important to achieve maximum reliable throughput of plasma, without trauma to the blood on the one hand or creation of a sensitive or unstable plasmapheresis procedure on the other. Further benefits can then be derived in terms of the efficiency of plasma extraction, possible reduction of the cost of the expensive filter membrane, and the amount of donor time that is required. The applicant departs entirely from the view that a controlled laminar flow must be established, with stratification of a plasma rich layer at the membrane surface, and with outward radial migration of cellular matter in the blood. Instead, applicant induces a strong vorticity in the form of successive, alternately circulating, annuli about the spinner and occupying the gap between the spinner and the shear wall. This vortex action is of a type, referred to as Taylor vortices, first proposes by G.I. Taylor in 1923 and described by him in Phil. Trans. Am., Vol. 233, pp. 289-293 in "Stability of a Viscous Liquid Contained between Two Rotating Cylinders." Prior theoretical and computer simulation studies of the Taylor phenomenon (of which there are many) posit that the flow that is created in a Couette structure, under proper conditions, establishes a continuous sequence of annular vortex cells along the longitudinal axis of the cylinder. As seen in the fragmentary and idealized views of Figs. 2 and 3, which are not to scale, each cell has a circulatory flow within the plane of a cross-section that is radial to the central (rotational) axis of the spinner, with the direction of circulation alternating between successive cells. The perspective view of Fig. 2 depicts the generally helical flows within an individual cell and the counter-rotation of alternate cells within the series. The sectional view of Fig. 3 represents a computer generated approximation of flows within a cross-section extending along the spinner axis and at some radius relative to the axis.

The great majority of prior studies, however, have been of stable liquid systems maintained under constant operating conditions. Proposals for practical utilization of the effect have heretofore been limited, although the theoretical investigations have been and remain extensive in character.

The Taylor number, as it is now called, was defined by G. I. Taylor as the product of the Reynolds number and the square root of the gap between the rotor and housing divided by the square root of the radius of the rotor. The vortices begin to appear, superimposed on the tangential flow induced by the relative rotation, when the Taylor number is greater than 41.3. Many of the investigations in the past have induced relative movement by spinning either the housing or the central mandrel, or both. In the examples given hereafter, only the central mandrel is spun, although the filter membrane 40 may be disposed on the spinner 22 surface as well as on the stationary cell. It is also feasible to utilize the vortex action and other flow conditions in a variety of other configurations and with other media, as discussed below.

An important consideration, is that the entire useful surface of the membrane 40 is made to contribute to the extraction process even though the suspension changes constantly because of filtrate extraction. The vortex action is preferably augmented to the level at which the Taylor number is in excess of 70, and more preferably in excess of 100, but usually not greater than about 250, throughout the length of the filter membrane despite the substantial increase in viscosity as plasma is extracted. Because the vortex cells fill the radial gap and sweep the membrane surface in closely tangential relationship, velocity and force components of substantial magnitude adjacent the membrane 40 surface are induced that are orthogonal to the forces induced by rotation of the spinner 22. This circulating motion, coupled with convection along the spinner 22 axis, constantly seeks to remove any adherent cells from the surface of the membrane 40 and replenishes available plasma for filtration through the membrane pores. Any given point on the membrane 40 is swept in a time varying fashion by matter moving in alternately parallel and anti-parallel directions relative to the axis of rotation of the spinner. The circulatory forces that exists thus supplement the shear forces exerted on the blood by viscous drag, tangential to the spinning membranes 40 surface.

At the same time, as seen in Fig. 3, constant interchanges between adjacent cells take place, for both plasma and cellular components, although the plasma probably is transported longitudinally more readily than is the cellular matter. The interchange tends to substantially diminish any hematocrit gradient across the gap adjacent the spinner 22, although one can observe a color gradient increasing in intensity as one travels from the inlet to the outlet. Nonetheless the system achieves the desired effect of utilizing all incremental areas of the entire spinner 22 with substantially equal efficiency. Because the vortex cells are not static but are constantly moving downardly toward the outlet 52, any given incremental area on the membrane is sequentially exposed to different vortex forces, militating against tendencies toward buildup of cell deposition. The scrolling motion of the vortex cells as the blood mass moves causes the vortex cells to be angularly disposed or slanted relative to the central axis.

The circumferential rotation within the Taylor vortex cell must not impart so high a velocity that movement inwardly toward the rotating spinner impels red cells toward the membrane with sufficient velocity to induce cell deposition on the membrane. On the opposite side, impingement of cells against the stationary outer wall cannot be so vigorous as to induce damaging turbulence. Both of these conditions can occur with strong vortex action within a range of acceptable shear, the consequences on the one hand being clogging of the pores of the membrane with a concomitant increase in transmembrane pressure and a reduction of plasma flux, and on the other the introduction of cell damage and hemolysis.

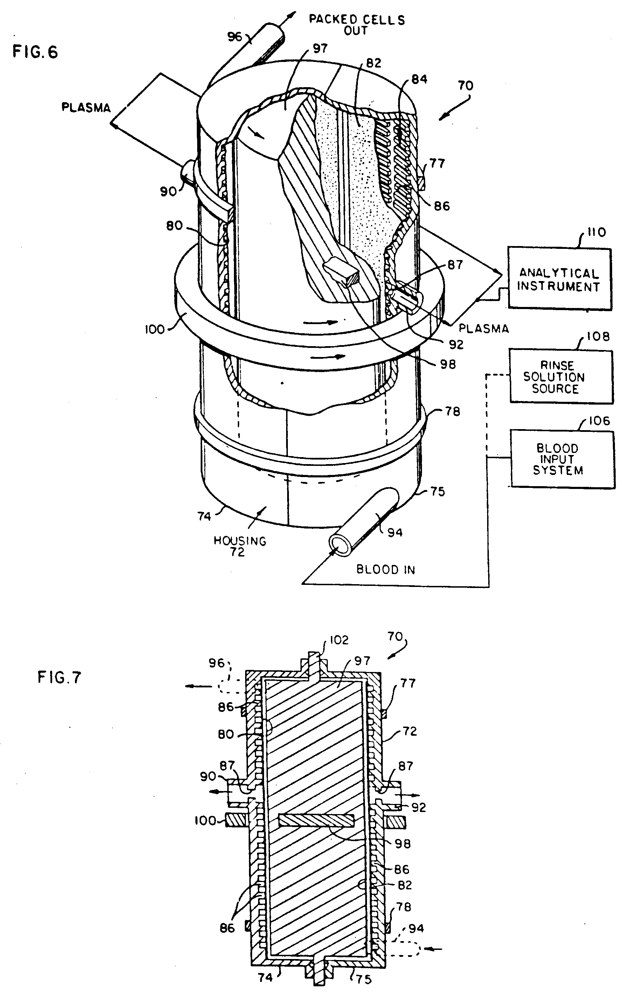

Confirmation of the existence of the vortex cells has been derived in several ways. In contradistinction to the "shear centrifugation" theory substantial plasma fluxes or throughputs have been attained utilizing a membrane disposed at the stationary shear wall as described in conjunction with Figs. 6 and 7. It is evident that no significant plasma extraction would result if the entire mass were centrifuged forcefully enough for cells to migrate radially outwardly and to pack against the outer surface. Also, although the spinner mass appears to be uniform when viewed through a transparent shear wall by the naked eye, the use of a synchronized stroboscopic light and high speed flash photography clearly reveals the existence of the vortex cells. Under stroboscopic light, the vortices appear as in photographs depicted in the prior art, as in Fig. 7 of an article by J. E. R. Coney et al entitled "A Study Of Fully Developed, Laminar, Axial Flow And Taylor Vortex Flow By Means Of Shear Stress Measurements," in O.-Mech. Eng. Sci., Volume 21, No. 1, 1979, pp. 19-24. Further, the vortex cell formation becomes even more visible when the separator is caused to function with a mixture of minute reflection crystals in water. Experiments were also conducted in which suspended matter, in the form of hollow micro-beads, were passed through the separator mechanism in a water suspension. The system readily filtered the heavier water through the membrane, which again would not have taken place had there been a stratification of the heavier liquid outside the lighter particle matter. The theoretical existence of vortices is thus confirmed by a variety of direct evidence.

Enhancement of the effectiveness of the vortex action and concurrent reduction of traumatic incidents arises from selective control of surface characteristics of the membrane 40. The sweeping tangential flow in a vigorous vortex as practiced herein brings fragile red cells (in a blood flow) into intimate but tangential relation to the membrane surface. Although commercially available membranes appear solid and feel smooth (at least on one side) their submicron characteristics can interact with much larger matter flowing across the surface. It is therefore preferred, in this plasmapheresis system, to employ a membrane having surface perturbations whose order of magnitude is smaller than the pore size. While a pore size of about 0.9 microns, for example, inhibits passage of red cells, a mean variation at the surface of less than that pore dimension assures a much greater freedom from capture or damage of the cells. A series of analyses using different membranes confirms that hemolysis (evidenced by increasing greater red coloration) increases in rough proportion to surface irregularities. In addition blockage by entrapment of cells appears as an increase in transmembrane pressure. While membranes can vary greatly in thickness (e.g. from 10 to 20 microns), surface protuberances and cavities should not exceed the stated relationship to pore size where blood is the medium.

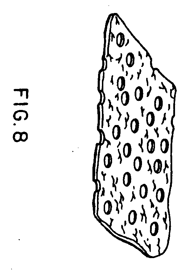

A suitable membrane 40 is depicted in Fig. 8 as recorded on an electron micrograph. This specific membrane is a "Nuclepore" (Trade Mark) polycarbonate membrane; another often suitable membrane is the "Gelman polysulfone 650" (Trade Mark), but the surface characteristics of this product appear to vary, perhaps because of the use of a surface surfactant. In contrast, a nylon membrane (e.g. "Cuno 66" (Trade Mark) produced by AMF) appears smooth and feels slippery to the touch but when examined at substantial magnification has a complex submicron surface pattern of protrusions and concavities. This type of surface is believed to generate local increases in shear stress and consequently to damage and entrap red blood cells to an extent which is evident on visual inspection. Furthermore this particular irregular surface demonstrably acts to activate platelets which cumulatively interlock and build up a barrier on the membrane, blocking the pores.

The character of the membrane surface is also important from another aspect, because if surface activation of cells commences during filtration, it continues when the rotor is stopped as during a return cycle. Consequently during the time of use of a disposable there can be a substantial loss of porous surface and marked increase in transmembrane pressure.

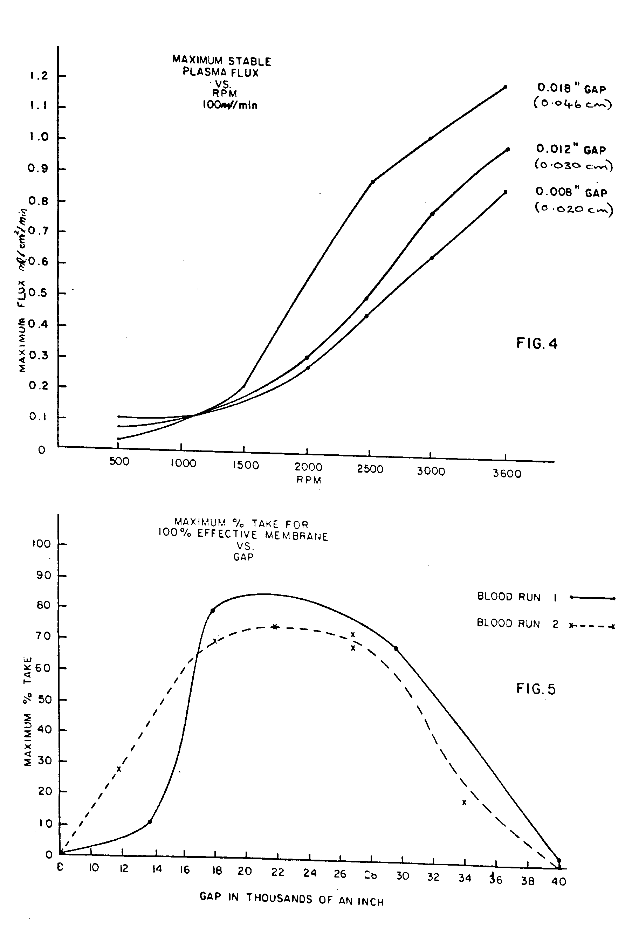

Increases in plasma flux, with respect to increase in shear, may be seen from Fig. 4, which depicts the maximum stable plasma flux in relation to angular velocity for a given blood flow (100 ml/min). The curves represent the variations encountered as angular velocity is increased for different gap sizes under the otherwise stated conditions of gap, rpm and diameter. Up to certain levels the rate of increase is Substantially linear, but thereafter the rate of increase follows a steeper slope. In point of fact, the region at which the rate of increase in plasma flux rises to a higher slope can now be identified as the region of onset of the Taylor vortices. Using the previously stated Taylor equation to determine the onset of vortex cells, Taylor flow begins in the 1100-1250 r.p.m. range for the 0.018" (0.046cm) gap, at 2600 r.p.m. for the 0.012" (0.030cm) gap and at above 4600 r.p.m. for the 0.008" (0.020cm) gap. Although the shear rate of the smaller gap is substantially higher, so that plasma flux should theoretically be greater if shear rate is the predominant factor, this is the case only where the rpm's are low enough to be below the Taylor instability for the gaps shown in Fig. 4. Below 1000 r.p.m., for example, the smaller gap has superior plasma flux. In contrast, the larger gap provides significantly more plasma flux, by a factor of several times, when vortex cells are properly established and have amplitudes that fill the gaps and provide vigorous cell Circulation. In preferred examples, present systems operate at Taylor number in the range of 180-200.

Donnelly et al in "Experiments On The Stabiity Of Viscous Flow Between Rotating Cylinders, VI, Finite-Amplitude," Proc. Ray Soc., London, Volume 283, 1965, pp. 531-546 established that the amplitude of the Taylor vortex varies as the square root of the difference between the Taylor number at the operating rpm and the critical Taylor number. Donnelly et al, however, used a slightly different formulation for the Taylor number where the Taylor number is proportional to the square of the rpm, so that a direct comparison to values derived from the previously stated equation are not feasible. Nevertheless_{,} the amplitude of the vortex cells increases with the Taylor number, the cells forming first at the rotating wall and expanding outwardly to fill the radial gap. When the vortex cell substantially fills the gap the action of viscous drag at the spinner surface provides local circumferential forces that are much greater than comparable forces at the stationary outer wall. The vortex cell circulation provides sweeping movement in the orthogonal direction at both walls, and it appears that this also is greater at the moving wall than the stationary wall. Vortex cell circulation at the outer wall can be increased to the level originally existing at the inner wall by increasing the rotor rpm. Either or both of the walls can include a filter membrane to achieve greater filtration efficiency than flat plate and other prior systems.

Because the amplitude of the Taylor vorticity increases more quickly than the shear rate as the rpm increases, the beneficial effects of Taylor vorticity provide significant contribution to the high increase in plasma flux. Relatively larger size gaps provide an increase in Taylor vortex amplitude from a lower rpm threshold region and consequently a stronger vortex action at an acceptable shear rate. There is, however, a limit at which vorticity overpowers shear, with detrimental effects. This is primarily due to the radial inward tangential forces exerted during strong vortex action, which tend to cause cell deposition on the moving membrane. When this occurs, there is a decrease in the plasma flux, and an increase in the transmembrane pressure. As illustrated in Fig. 5, the use of a large gap size, 0.040" (0.10cm), results in just such a diminution of performance. Fig. 5 shows variations in percentage take relative to gap size, given a rotational rate of 3600 r.p.m. It can be seen, from this Figure, that chracteristics drop off substantially at gap sizes in excess of 0.030" (0.076cm). Clogging of the membrane requires the system to attempt to force filtration and substantially increases problems with cell damage.

A different limiting aspect is the cell disruption which can occur if vortex action is too strong, by virtue of radially outward movement of cells with such velocity that they impinge on the stationary wall, to cause hemolysis. This apparently, however, will occur subsequent to the cell deposition problem in most instances.

Hemolysis is to be avoided or kept to a minimum in plasmapheresis systems in accordance with the invention, but it must be recognized that there is no strictly defined value or limit as to the amount of permissible cell damage. Objective operative criteria, such as the shear level, are not precise determinants of whether an unacceptable level of hemolysis will be introduced in these dynamic systems. Shear limits for flat plate devices were previously considered to be in the range of 7500 sec, but present systems using vortex cell action have operated without significant hemolysis at in excess of 12,000/sec.

The same principles of vortex action are utilized in conjunction with the configuration of the present application, as depicted in Figs. 6 and 7, to which reference is now made. This comprises a separator device 70 for diagnostic and other analytical applications, wherein small samples may be taken and separated into constituents for individual analysis. The device of Figs. 6 and 7 also pertains to blood separation, and the usage of separated plasma or serum, although in a diagnostic procedure. However, the principles of construction and operation will be recognized by those skilled in the art as being applicable to other separated constituents and to entirely different liquid suspensions as well.

In the separator device 70 of Figs. 6 and 7, the cylindrical housing 72 is constructed as a separable member so that access can be had to the interior. In this example the housing 72 is formed as a pair of split halves 74, 75 having integral top and bottom end portions providing a substantially enclosed cell when joined together. The split halves 74, 75 are held together by retainers, such as clamping rings 77, 78. Seals (not shown) may be disposed between the abutting surfaces of the split halves 74, 75 as long as the concentricity of the interior surface is maintained. The inner wall of each split half 74, 75 is defined by one or more removable filter membranes 80, 82 attached as by a strippable adhesive to the adjacent lands 86 on a network 84 of grooves in the inner surfaces of the housing, these groove networks 84 in each half 74, 75 providing communicating paths through radial orifices 87 for plasma or serum flow between the inner surface of the membrane 80 or 82 and an outlet port 90 or 92, respectively. The outlet ports 90, 92 for filtrate are connected together to provide effluent for fractionation or for analysis by associated instruments. As with the system of Fig. 1, the conduit system under each membrane 80 or 82 provides adequate interconnected flow area to avoid the introduction of substantial impedance to flow of filtrate to the appropriate outlet port 90, 92. The membranes 80, 82 may be removed and replaced by detaching them from the adhesive backing, which need not be strong in view of the fact that the housing 72 and membranes 80, 82 are stationary. However, the adhesive may also be dissolved by chemical means and a new adhesive applied, or a mechanical attachment structure may alternatively be utilized as long as the concentricity of the inner housing face is maintained.

The whole blood inlet 94 for this structure is coupled tangentially to the housing inner wall at a lower region of the housing, while the outlet 96 is positioned tangentially to the inner wall adjacent an upper end of the housing 72. Within the housing 72 is mounted a cylindrical spinner 97, having an internal magnetic member 98 mounted intermediate its ends. The spinner may be a smooth, plated surface member having an outer diameter providing the chosen gap dimension relative to the inner wall of the housing 72 defined by the membranes 80, 82 and interconnecting housing wall segments. The end surfaces of the spinner 97 are also spaced apart from the end surfaces of the housing 72 by a predetermined amount. The entire housing, in its mid region, is encompassed by a rotatable magnetic drive 100 arranged in operative relation to the magnet 98 within the spinner 97. The drive is positioned with a slight vertical displacement from the magnetic element 98, so as to tend to bias the spinner 97 upwardly and reduce the force of gravity acting against the bottom end wall of the housing. End bearings 102, 103 support the spinner 97 for rotation about the central axis.

A blood input system 106, which may comprise not only a source of whole blood but also a source of anticoagulant and saline solution if desired is coupled to the blood input to the system. A source of a rinsing solution 108 is alternatively coupled to the same input 94, the rinsing solution being substituted manually or automatically for the blood input. Plasma or serum filtered through the system is passed from the outlet 96 to an analytical instrument 110. Typically, the whole blood sample need only be sufficient in size to enable a period of stable extraction of filtrate for a long enough time to obtain an adequate plasma or serum sample (typically in the range of 5 to 30 milliliters).

The operation of the system with whole blood input is again based upon establishment of enhanced vortex flow throughout the entire length of the filter membranes 80, 82. To this end, the magnetic drive 100 synchronously rotates the inner spinner 96 through its magnetic coupling with the magnetic element 98 at a rotational velocity in the range of 3600 r.p.m., it being assumed that the spinner again is - approximately 1" (2.54cm) in diameter. Using a gap of .018 to .030" (0.046 to 0.076cm), suitably adjusted for blood viscosity and other conditions, vortices are created that fill the radial gap between spinner 97 and housing 72. The existence of vigorous vortices that entirely fill the gap is more critical when the membrane surface is static than in the example of Fig. 1, Because the vortices start near the spinner surface and grow outwardly until they sweep the outer wall it is desirable to insure that viscous damping losses at the stationary wall do not prevent suitably vigorous vortex action at the outer surface. Thus the Taylor number is increased 5-10% over the values previously given for the Fig. 1 system, as by increasing the rotational speed. No hemolysis is observed when this change is made. The centrifugal displacement effects imparted by the rotation of the inner spinner 97 that tend to deposit cellular matter and other heavier matter on the surface of. the membranes 80, 82 are overcome by the sweeping vortex motion at the membrane surface.

Practical systems in accordance with this example have achieved plasma filtration rates with average hematocrit (38-44% blood) far in excess of rates achieved by the best parallel plate technology previously known. The plasma flux for stable output without evidence of pore clogging was some 5-10% less than the 70% take and 39-43 ml/min rate achieved by the system of Fig. 1 for a 1" (2.54cm) spinner. Plasma throughput was maintained without substantial diminution in properties during the extraction of 2-3 units (500-750 ml) of plasma. The plasma take is stable and amenable to achieving higher throughput by use of higher spin rates or other variables. The effective membrane area can be relatively increased because the membrane being stationary needs only enough supporting surface to be held concentric. Consequently, this System demonstrates further that the vigor of the augmented vortex condition and the sweeping action imparted by the orthogonal flow components at the membrane surface, have not only established a new filtration approach using high shear but that it also incorporates a signficantly effective cleaning action.

With stationary membranes 80, 82 about the spinner 97, the system of Fig.6 and 7 can provide successive samples of relatively small amounts of filtrate from inputs provided via the whole blood system 106 from many different sources. In a diagnostic system, where the characteristics of the plasma are serum alone are of concern, contamination is not a problem and the surface cleaning effected by the vortex action can maintain high filtration efficiency for a substantial period of time. Alternatively, saline solution from the input system 106 can be provided between whole blood samples to effect some clearing of the system, or as a further alternative the membranes 80, 82 may be cleaned by use of rinse solution from the source 108. When filtration efficiency drops in unrecoverable fashion below a selected level, the system need only be stopped, the housing 72 emptied, and the housing 72 then opened and new filter membranes 80, 82 used to replace the previously used elements.

In the example of Figs. 6 and 7, passage of whole blood is from a lower input to a higher output, but the essential vortex action and scrolling advance of the vortex cells are unimpeded even though the net flow proceeds upwardly against gravity. As in the prior example the vortices do not remain fixed but translate upwardly in continuous fashion, thus constantly sweeping incremental areas of the surface of the filter membranes.

A number of other variations of this system, including its use for the concentration of red blood cells or platelets, will present themselves to those skilled in the art. The separator device may be fabricated as a low cost disposable, for diagnostic or conventional plasmapheresis applications. The housing structure for a disposable unit dealing with small blood samples may be configured so as to provide a retainer chamber for the packed cell output, enabling the unit, including the waste blood, simply to be disposed of following collection of the needed amount of filtrate.

## Claims

1. A system for filtering a liquid suspension, comprising a housing (72) having an inner surface carrying a filter membrane, and a rotor (97) mounted for rotation within the housing and having an outer surface which together with the inner surface of the housing defines a gap to receive the liquid suspension, characterized in that the housing (72) is constructed of separable parts (74, 75), each having two end portions, and the end portions providing end bearings (102) for the rotor, and retaining means (77,78) releasably holding the parts together to form a substantially enclosed cell, the inner surface of the housing has a network (84) of surface grooves, each of the separable parts (74, 75) has a corresponding filter membrane (80, 82) releasably attached to its inner surface and covering the surface grooves, and a passage is provided in the housing which communicates with the surface grooves when the separable parts are joined and thus receives the filtrate passing through the filter membrane.

2. A system as claimed in claim 1 in which the membrane surface is smooth with deviations no greater than the pore size of the membrane.

3. A system as claimed in claim 1 or 2 in which the membrane pore size is in the range of 0.1 to 1.0 microns.

4. A system as claimed in claim 3 in which the membrane pore size is in the range 0.8 to 1.0 microns.

5. A system as claimed in claim 1, 2, 3 or 4 including drive means magnetically coupled to the rotor through the housing.

6. A system as claimed in any of claims 1 to 5 in which the width of the gap is 0.046 to 0.076 cm. (0.018 to 0.030 inch).

7. A system as claimed in claim 6 in which the diameter of the rotor is approximately 2.5 cm (one inch).

8. A method of filtering a liquid suspension using the system of claim 1 in which the dimensions of the gap and the speed of rotation of the spinner are chosen so that Taylor vortices are formed in the gap throughout the entire length of the filter membranes with the Taylor number being in the range of 100 to 250.

9. A method as claimed in claim 8 in which the Taylor number is in the range 180 to 200.

10. A method as claimed in claim 8 or 9 in which the liquid suspension is blood.

## Patentansprüche

1. System zum Filtern einer Flüssigkeitssuspension umfassend
- ein Gehäuse (72) mit einer Innenoberfläche, die eine Filtermembran trägt, und
- einen Rotor (97), der für eine Drehbewegung innerhalb des Gehäuses montiert ist und eine Außenoberfläche aufweist, die zusammen mit der Innenoberfläche des Gehäuses einen Spalt bildet, um die Flüssigkeitssuspension aufzunehmen,
dadurch gekennzeichnet,
daß das Gehäuse (72) aus trennbaren Teilen (74, 75), die jeweils zwei Endbereiche haben, wobei die Endbereiche Endlager (102) für den Rotor bilden, und Halteeinrichtungen (77, 78) aufgebaut ist, die die Teile lösbar zusammenhalten, um eine im wesentlichen abgeschlossene Zelle zu bilden,
daß die Innenoberfläche des Gehäuses ein Netzwerk (84) von Oberflächennuten aufweist,
daß jedes der trennbaren Teile (74, 75) eine entsprechende Filtermembran (80) aufweist, die lösbar an ihrer Innenoberfläche angebracht ist und die Oberflächennuten überdeckt, und
daß eine Passage in dem Gehäuse vorgesehen ist, die mit den Oberflächennuten in Verbindung steht, wenn die trennbaren Teile verbunden sind, und somit das Filtrat erhält, welches durch die Filtermembran hindurchgeht.

2. System nach Anspruch 1,
bei dem die Membranoberfläche glatt ausgebildet ist mit Abweichungen, die nicht größer sind als die Porengröße der Membran.

3. System nach Anspruch 1 oder 2,
bei dem die Membran-Porengröße im Bereich von 0,1 bis 1,0 »m liegt.

4. System nach Anspruch 3,
bei dem die Membran-Porengröße im Bereich von 0,8 bis 1,0 »m liegt.

5. System nach Anspruch 1, 2, 3 oder 4,
das eine Antriebseinrichtung aufweist, die mit dem Rotor durch das Gehäuse magnetisch gekoppelt ist.

6. System nach einem der Ansprüche 1 bis 5,
bei dem die Breite des Spaltes 0,046 bis 0,076 cm (0,018 bis 0,03 Inch) beträgt.

7. System nach Anspruch 6,
bei dem der Durchmesser des Rotors ungefähr 2,5 cm (1 Inch) beträgt.

8. Verfahren zum Filtern einer Flüssigkeitsuspension unter Verwendung des Systems nach Anspruch 1, bei dem die Dimensionen des Spaltes und die Drehzahl der Schleuder so gewählt sind, daß über die gesamte Länge der Filtermembranen Taylor-Wirbel in dem Spalt gebildet werden, wobei die Taylorzahl in dem Bereich von 100 bis 250 liegt.

9. Verfahren nach Anspruch 8,
bei dem die Taylorzahl im Bereich von 180 bis 200 liegt.

10. Verfahren nach Anspruch 8 oder 9,
bei dem die Flüssigkeitssuspension Blut ist.

## Revendications

1. Système de filtration d'une suspension liquide, comprenant un boîtier (72) présentant une surface intérieure portant une membrane de filtre, et un rotor (97) monté à rotation dans le boitier et présentant une surface extérieure qui, conjointement à la surface intérieure du boîtier, définit un intervalle pour recevoir la suspension liquide,
caractérisé en ce que le boîtier (72) est construit sous forme de parties séparables (74, 75), dont chacune a deux parties d'extrémités, les parties d'extrémité formant les paliers d'extrémité (102) du rotor, et des moyens de maintien (77, 78) retenant de façon amovible les parties ensemble pour former une cellule sensiblement fermée, la surface intérieure du boitier présente un réseau (84) de rainures de surface, chacune des parties séparables (74, 75) a une membrane de filtre correspondante (80, 82) fixée de façon amovible à sa surface intérieure et couvrant les rainures de surface, et un passage est formé dans le boîtier qui communique avec les rainures de surface quand les parties séparables sont réunies et reçoit ainsi le filtrat passant à travers la membrane du filtre.

2. Système suivant la revendication 1, dans lequel la surface de la membrane est lisse, avec des écarts non supérieurs à la dimension de pore de la membrane.

3. Système suivant l'une quelconque des revendications 1 ou 2, dans lequel la dimension de pore de la membrane est de l'ordre de 0,1 à 1,0 »m.

4. Système selon la revendication 3, dans lequel la dimension de pore de la membrane est de l'ordre de 0,8 à 1,0 »m.

5. Système suivant l'une quelconque des revendications 1 à 4, comprenant des moyens d'entraînement magnétique couplés au rotor à travers le boitier.

6. Système suivant l'une des revendications 1 à 5, dans lequel la largeur de l'intervalle est 0,046 cm à 0,076 cm (0,018 à 0,030 pouce).

7. Système suivant la revendication 6, dans lequel le diamètre du rotor est environ 2,5 cm (un pouce).

8. Procédé de filtration d'une suspension liquide utilisant le système de la revendication 1 dans lequel les dimensions de l'intervalle et la vitesse de révolution du rotor sont choisies pour que des vortex de Taylor se forment dans l'intervalle sur toute la longueur de la membrane filtrante avec un nombre de Taylor compris dans la plage de 100 à 250.

9. Procédé selon la revendication 8 dans lequel le nombre de Taylor est compris dans la plage de 180 à 200.

10. Procédé selon l'une quelconque des revendications 8 ou 9, dans lequel la suspension liquide est du sang.
